## Europäisches Patentamt

## European Patent Office

## Office européen des brevets

(11) Publication number: **0 310 619 B1**

## EUROPEAN PATENT SPECIFICATION

(45) Date of publication of patent specification: **06.04.94**

(51) Int. Cl.5: **C12N 15/70**, C12N 15/74, C12P 21/02

(21) Application number: **87904076.4**

(22) Date of filing: **23.06.87**

(86) International application number:
**PCT/GB87/00440**

(87) International publication number:
**WO 87/07909 (30.12.87 87/29)**

(54) **LIGHT INDUCIBLE PROMOTER.**

(30) Priority: **23.06.86 GB 8615263**

(43) Date of publication of application:
**12.04.89 Bulletin 89/15**

(45) Publication of the grant of the patent:
**06.04.94 Bulletin 94/14**

(84) Designated Contracting States:
**AT BE CH DE FR GB IT LI LU NL SE**

(56) References cited:

**Proc. Natl. Acad. Sci. USA, vol. 84, april 1987, J.M. Balsalobre et al., pp. 2359-2362**

**Proc. Natl. Acad. Sci. USA, vol. 81, Sept. 1984, L. Kroos et al. pp. 5816-5820**

**Proc. Natl. Acad. Sci. USA, vol. 80, March 1983, L.J. Shimkets et al., pp. 1406-140**

(73) Proprietor: **UNIVERSITY OF WARWICK
Gibbet Hill Road
Coventry, Warwickshire CV4 7AL(GB)**

(72) Inventor: **HODGSON, David, Alan
14 Lynchgate Court
Cannon Park
Coventry Warwickshire CV4 7EH(GB)**

(74) Representative: **Dean, John Paul et al
Withers & Rogers
4 Dyer's Buildings
Holborn
London EC1N 2JT (GB)**

Chemical abstracts, vol. 103, 1985, ( Columbus, Ohio, US), T. Furuichi et al., p. 189, abstract 190877g, & J. Bacteriol. 1985, 164(1), p. 270-5

Science, vol. 230, 4 Oct. 1985, M. Dworkin et al., pp. 18-24

Chemical Abstracts, vol. 106, 1987, (Columbus, Ohio, US), A. Martinez-Laborda et al., s. abstract no. 13804x, & MGG, Mol. Gen. Genet. 1986, 205(1), 107-14

Nature, vol. 310, no. 5973, July 1984, (London, GB), L. Herrera- Estrella et al., pp. 115-120

## Description

This invention relates to a light-inducible promoter derived from myxobacteria, and especially but not exclusively Myxococcus xanthus, for use in controlling the expression of desired genes in prokaryotes such as M.xanthus or Escherichia coli. M.xanthus secretes potentially useful enzymes and antibiotics and, as it can be cultured under standard industrial conditions is useful as an organism for the industrial production of desired polypeptides. The newly-identified and isolated light-inducible promoter enables commercially useful genes put under its control to be switched on or off at will by the introduction of a light source.

M.xanthus is the genetically best characterised of the myxobacteria (Rosenberg, 1984). These Gram negative, pigmented bacteria move by gliding and when starved form spores (which are resistant to heat, dessication and X-rays) in elaborate multicellular complexes termed fruiting bodies. The spores can initiate new colonies when conditions improve. M.xanthus also produces an extensive array of extracellular enzymes and antibiotics, one of which antibiotics is now undergoing trials as a localised wide-range chemotherapeutic agent. Also, M.xanthus has been proposed as a development model for higher organisms. The advantage that bacterial genetics, both classical and genetic engineering based, can bring to the study of development is amply illustrated in this bacterium. A number of transducing phages are available for the exchange of DNA between different strains. The fortuitous property of coliphage P1 to inject its DNA into M.xanthus without being able to replicate has been exploited to introduce transposons into M.xanthus and plasmids that have been packaged in vivo in E.coli (Dworkin and Kaiser, 1985),

We have discovered a number of colony colour mutants present in the M.xanthus strain collection of Prof. Dale Kaiser at the Stanford University Medical Centre, Stanford, California, CA94305, U.S.A. One of these mutants, strain DK2727, produced orange colonies as a result of transposon Tn5 insertion. Tn5 is a bacterial transposon whose expression confers Kanamycin resistance. It was found that DK2727 produced carotenoids in both light and dark, and that insertion of the Tn5 had not destroyed a structural gene to give the orange colony colour but rather had activated the gene that controls carotenoid production which is under the control of a light-inducible promoter.

This light-inducible promoter has now been isolated and introduced into a cloning vehicle in such a way that a desired gene can be introduced adjacent to the promoter so that the promoter can control the expression of that gene.

In broad outline the experimental work that resulted in the present invention can be summarised as follows:

Firstly, it was already known that M.xanthus produces orange carotenoid pigments when exposed to bright light. These pigments protect the bacteria as in their absence the cells lyse. The mutant DNA from the DK2727 strain of M.xanthus, which encodes the production of carotenoids in the dark as well as the light as a result of the Tn5 insertion, was cloned out of the mutant strain.

We deduced that the carotenoid gene in the normal strains was probably under the control of a light-inducible promoter. The equivalent gene was then cloned out of a normal M.xanthus cell. We then identified the promoter or controlling region of the normal gene and inserted it into a cloning vector to place under the control of the promoter a promoterless gene having an easily assayable expression product. We were then able to establish that the gene was turned on only when the organism was exposed to light. It has also now been deduced that the promoter is turned on and off by means of a repressor system, the repressor being capable of deactivating the gene only in the absence of light.

At the thirteenth International Conference on the Biology of Myxobacteria held in Granada, Spain on 23-26th June, 1986, an informal agreement was reached as to the nomenclature of the genes concerning carotenoid synthesis. The Ω1911 region - which is the chief concern of this patent application - contains the carR gene(s). Three other, probably structural, genes, have also been identified which were termed carA, carB and carC. carA and carB are linked but distinct and mutants in the genes were present in strains MR7, and strains MR134 and DK2836 respectively (Martinez-Laborda et al. (1986)). carC has only recently been identified by the isolation of a Tn5-lac (Kroos and Kaiser (1983) insertion in strain MR403 (Balsalobre et al. (1987). carC maps to a part of the chromosome distinct from both carR and the carA-carB cluster. The recently submitted paper of Balsalobre et al. (1987) reports the insertion of a Tn5-lac into the carB locus (MR401) such that light controls beta-galactosidase expression. Other experiments have demonstrated that the carB locus is under the positive control of the carR locus. Strains unable to produce carotenoids under any conditions are referred to as Car⁻ (i.e. carotenoid lacking) whilst those producing carotenoids constitutively are referred to as Car$^c$.

According to one aspect of the invention there is provided a recombinant DNA sequence comprising a light inducible promoter sequence which is the light inducible promoter sequence of plasmid pDAH217 (NCIB12269) or pDAH201 (NCIB12493) or mutants of that sequence which function as a light inducible promoter.

As used herein the term "light-inducible promoter derived from a myxobacterium" includes light-inducible promoters derived from the native promoter sequence including mutants thereof and sequences which hybridise thereto.

Preferably, the DNA sequence comprises a restriction site in which a second DNA sequence which is to be expressed can be placed under the control of the said promoter.

The said restriction site is preferably one that occurs only a few times or more preferably only once in the DNA sequence.

Preferably, the said promoter is adjacent a desired structural gene which is under the control of the said promoter so that expression of said gene can be turned on by exposure to light. The desired gene may be a bacterial (e.g E.coli) or fungal or animal or plant gene.

It may be a mxyobacteria gene that in nature is not under the control of the light-inducible promoter.

The invention will now be described in more detail by way of example, and with reference to the accompanying drawings, which are summarised at the end of this specification.

## STRAINS AND MEDIA

The strains used are listed in Table 1.

Myxococcus xanthus was grown at 33°C in CTT Broth or on CTT Agar. CTT consisted of 1% (w/v) Difco Bacto Casitone, 10 mM Tris HC1 pH 8, 0 mM $MgSO_4$ and 1 mM Potassium Phosphate buffer pH 7.6 (Hodgkin and Kaiser, 1977). It was solidified with 1.5% Agar to form plates or with 0.7% to form CTT Soft Agar. E.coli was grown on Lenox broth or Lenox agar plates at 37°C unless otherwise stated (Maniatis et al., 1982).

## Cloning procedures

Restriction enzymes and DNA modifying enzymes were used as specified in the supplier's instructions. E.coli strain MC1061 (Casadaban and Cohen, 1980) was transformed by the Calcium chloride procedure as detailed in Maniatis et al. (1982). Unless otherwise stated the procedure for manipulation of E.coli was as specified in Maniatis et al. (1982).

### Conversion of DK2727 to DK6003

Transposons $Tn5$ ($Km^r$) and $Tn5$-132 ($Tc^r$) are both expressed in M.xanthus and can be introduced on the coliphage P1 (Kuner et al., 1981). Transposon Tn5-132 is a laboratory construct made by replacing the unique central region of $Tn5$, which specifies $Km^r$, with the $Tc^r$ determinant of transposon Tn10 (Berg et al., 1980). Unfortunately, the $Tc^r$ determinant is not easily selected for and $Km^r$ is the drug resistance determinant of choice. DK2727 has a transposon $Tn5$ inserted in its chromosome at a site termed $\Omega$1911, (a further explanation of the $\Omega$ terminology is given in Sodergren & Kaiser (1983)). Therefore, strains that contain $Tn5$ and which are going to be used for cloning experiments must first be converted to $Km^s$. This is done by the transposon replacement procedure of Kuner et al. (1981) as follows:

P1 phage containing Tn5-132 (P1 :: Tn5-132) was mixed with $4 \times 10^8$ of DK2727 cells at a multiplicity of infection of two in the presence of 5 mM CaCl. After 20 minutes of incubation at room temperature 3 ml of molten CTT Soft Agar was added and the mixture poured over a slant plate containing oxytetracycline.

The slant plate was prepared by pouring 20 ml of molten Agar containing 50 $\mu$g/ml oxytetracycline into a standard petri dish and allowing it to set at an angle. After returning the plate to the horizontal a further 20 ml of molten CTT Agar with no antibiotic addition was added as the second layer was set.

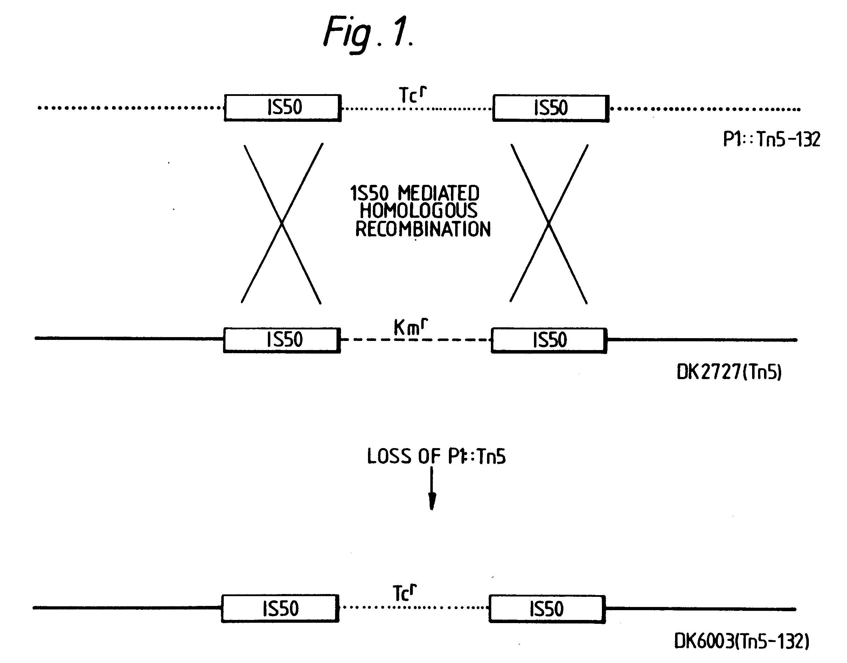

After five days incubation at 33°C a crescent of confluent growth was seen on the plate where the oxytetracycline had been at its lowest concentration. Independent colonies were found away from this confluent growth and these were picked and tested for loss of $Km^r$ and gain of $Tc^r$. This replacement occurred by the mechanism illustrated in figure 1. The slant plate system was used to ensure slow diffusion of the oxytetracycline into the Myxococcus cells which allowed the induction of the $Tc^r$ determinant of Tn5-132 before cell death. Several $Km^s.Tc^r$ cells were picked and one, DK6003, was chosen for the cloning procedure after Southern blot analysis had confirmed the predicted chromosomal structure.

Specialized transduction of plasmids from E.coli to M.xanthus using coliphage P1. (Modified from Shimkets et al., 1983)

A strain of E.coli catable of carrying a plasmid was grown to mid-exponential phase in L.B. plus 50 ug/ml Kanamycin. One half ml. of this culture was mixed with P1 :: Tn5-clr100 (which confers Cm$^r$) at a multiplicity of infection of ten and left to incubate at room temperature for twenty minutes. Five ml. of L.B. plus 10mM MgSO$_4$,50$\mu$g/ml. of Kanamycin and 12.5$\mu$g/ml of chloramphenicol were added and the culture was incubated overnight at 30°C with shaking. After dilution a mid-exponential phase culture,in the same medium and incubated at the same temperature,was then incubated at 42°C for 35 minutes to induce the phage. The culture was then incubated at 37°C for two hours. The culture was vigorously shaken throughout. Between 100 and 400$\mu$litres of the resultant phage lysate was mixed with one half ml. of late-exponential phase. M.xanthus in the presence of 5mM CaCl. After incubation at room temperature for 40 minutes, 3ml. of molten CTT soft agar was added and the mixture poured over a CTT agar plate containing 20$\mu$g/ml. of kanamycin. After incubation overnight the concentration of kanamycin in the plate was increased to 75$\mu$g/ml. by the addition of CTT soft agar containing a suitable concentration of the drug. Colonies formed from successful M.xanthus transductants were seen after a further four or five days incubation.

Cloning of DNA adjacent to the Ω1911 Tn5 insert (using the method of R.E. Gill,unpublished)

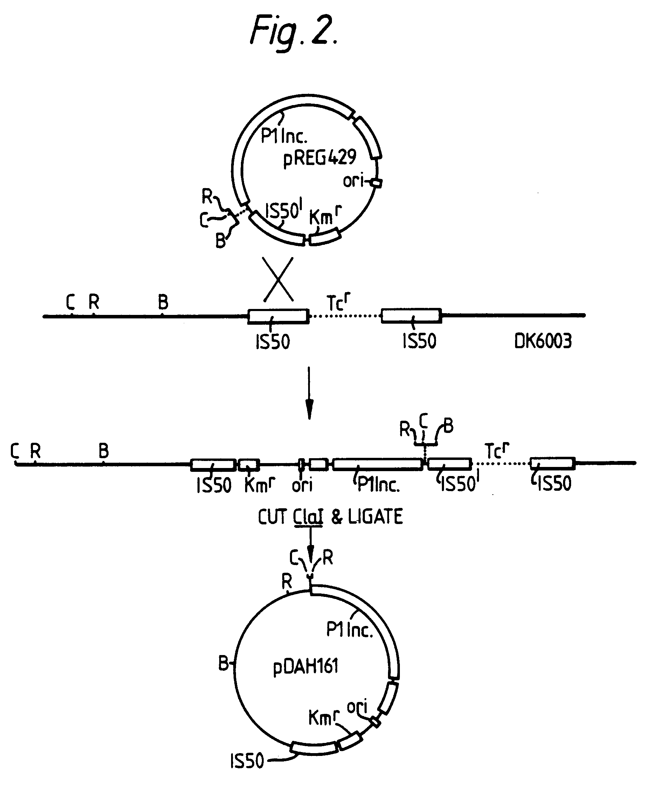

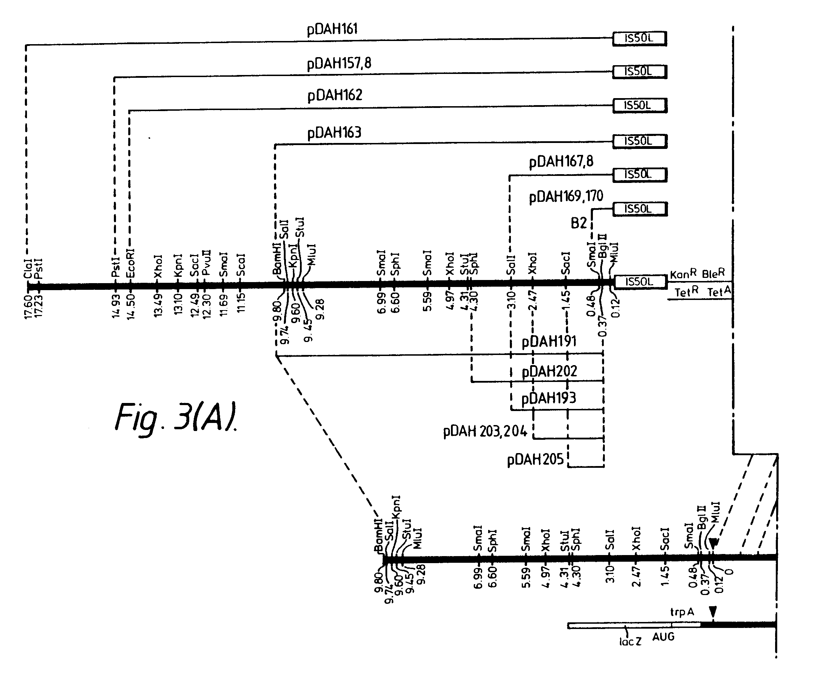

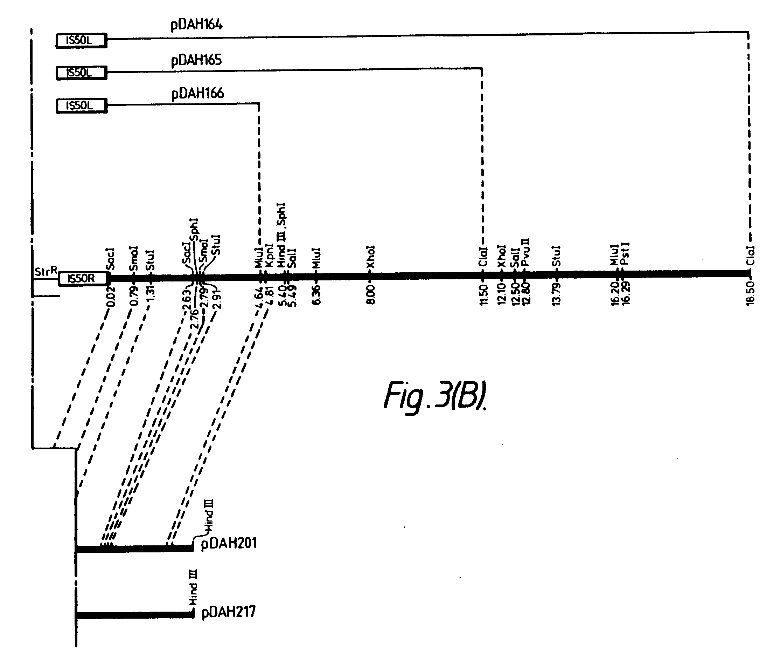

Strain DK2727 had been converted to a Km$^s$ and Tc$^r$ strain by the method described above. The resultant strain,DK6003, was used as a recipient of the plasmid pREG429 (see Fig.2). Plasmid pREG429 is freely available from the laboratory of Prof. Dale Kaiser supra. Plasmid pREG429 contains the P1 incompatability region of coliphage P1 which ensures its efficient transduction by that phage (Shimkets et al., 1983). pREG429 also contains an origin of replication; however, this only allows autonomous replication in E.coli thus pREG429 cannot replicate autonomously in M.xanthus, although the plasmid can be maintained in M.xanthus if it integrates stably into the chromosome. This chromosomal integration can be achieved in the case of pREG429 by homologous recombination between the slightly foreshortened IS50L (IS50') of pREG429 and either of the two IS50's of the Tn5-132 in DK6003. Figure 2 illustrates one of the two possible recombination events. The chromosomal DNA was purified from a pool of colonies of DK6003 into which pREG429 had been transduced. This pool must have contained examples in which the plasmid had inserted into each of the IS50's. The chromosomal DNA was cut separately with EcoRI,, ClaI or BamHI, diluted and ligated. Following transformation of E.coli strain MC1061 the plasmids pDAH161, pDAH162, pDAH163, pDAH164 and pDAH165 were obtained (Fig. 3) the origin of pDAH161 resulting from the cutting of chromosomal DNA with ClaI is illustrated in Figure 2 as an example of the origins of these five plasmids, pDAH162 resulted from the cutting of chromosomal DNA with EcoRI; pDAH163 resulted from cutting of chromosomal DNA with BamHI; pDAH164 and pDAH165 resulted from cutting of chromosomal DNA with ClaI. Plasmids pDAH164 and pDAH165 originated from chromosomes in which pREG429 had inserted into the right hand IS50 (as drawn in Figure 3) whilst pDAH161, pDAH162 and pDAH163 originated from left hand IS50 inserts.

Cloning of chromosomal DNA lacking the Tn5 insertion

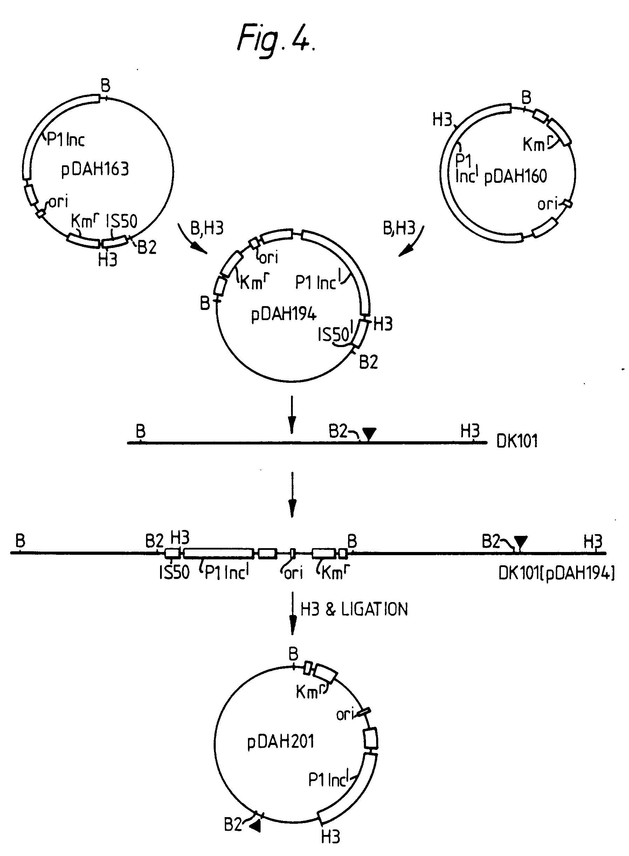

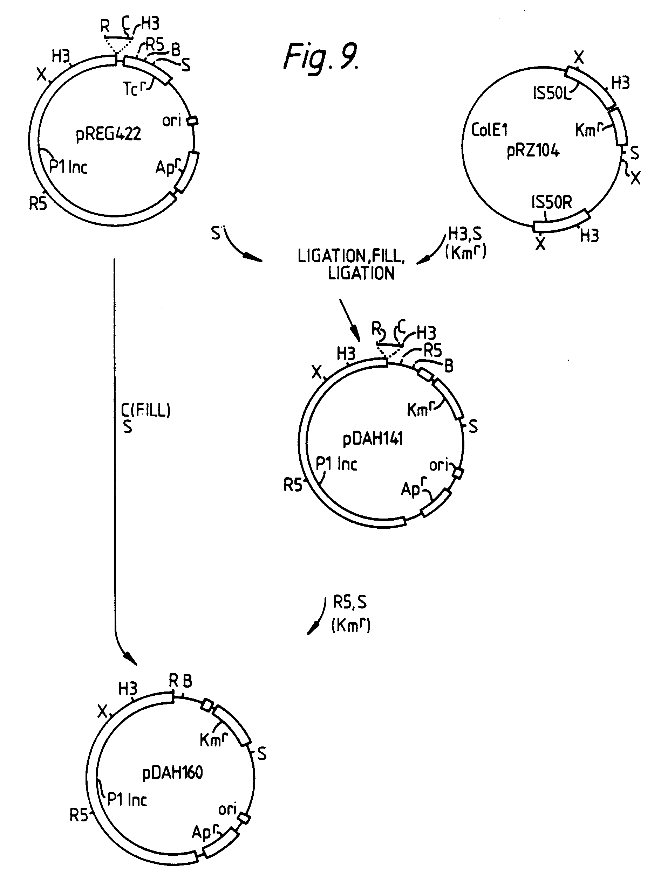

This was performed using a novel "reversing vector"- using plasmid pDAH160 which was derived from pREG422 (freely available from Prof. Dale Kaiser's laboratory) by elimination of the two Hind III sites from the latter plasmid. Figure 9 illustrates construction of pDAH160. The Hind III-SalI fragment containing the Km$^r$ determinant of pRZ104 (Jorgensen et al.1979) was ligated to SalI cut pREG422 to form pDAH141. To ensure ligation of the incompatible Hind III and SalI ends these were filled using Klenow fragment (Maniatis et al., 1982) after time had been allowed for Sal I - Sal I end ligation. The plasmid pREG422 is a derivative of pREG411 (Shimkets et al. 1983) in which one of the EcoRI sites has been deleted (R. E. Gill, personal communication). The EcoRV - Sal I fragment containing the Km$^r$ determinant of pDAH141 was inserted into pREG422 which had been cut with ClaI, filled using Klenow fragment and then cut with Sal I. The resulting plasmid, pDAH160, had unique EcoRI, BamHI, Hind III and XholI sites in such an orientation that myxobacterial DNA cloned into pREG429 could easily be inverted with respect to the vector sequences. Plasmid pDAH160 has restriction sites arranged such that chromosomal DNA cloned using pREG429 can be inverted with respect to the vector sequences. Plasmid pDAH163 was cut with BamHI and Hind III and the M.xanthus DNA isolated (see Fig. 4). This ligated into the large BamHI-Hind III fragment of pDAH160 to form pDAH194. This plasmid was transduced to DK101 and the chromosomol DNA was isolated from the

resultant co-integrate DK101 [pDAH194]. This chromosomal DNA was cut with Hind III, isolated and ligated. Following transformation the expected plasmid (pDAH201) which had resulted from complete Hind III digestion was identified and isolated.

Construction of the promoter fusion probe

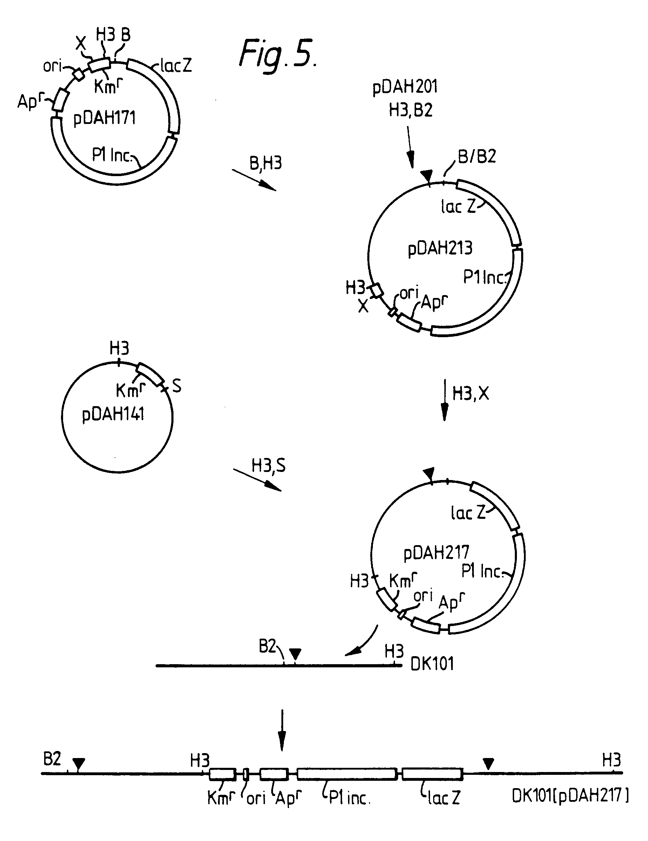

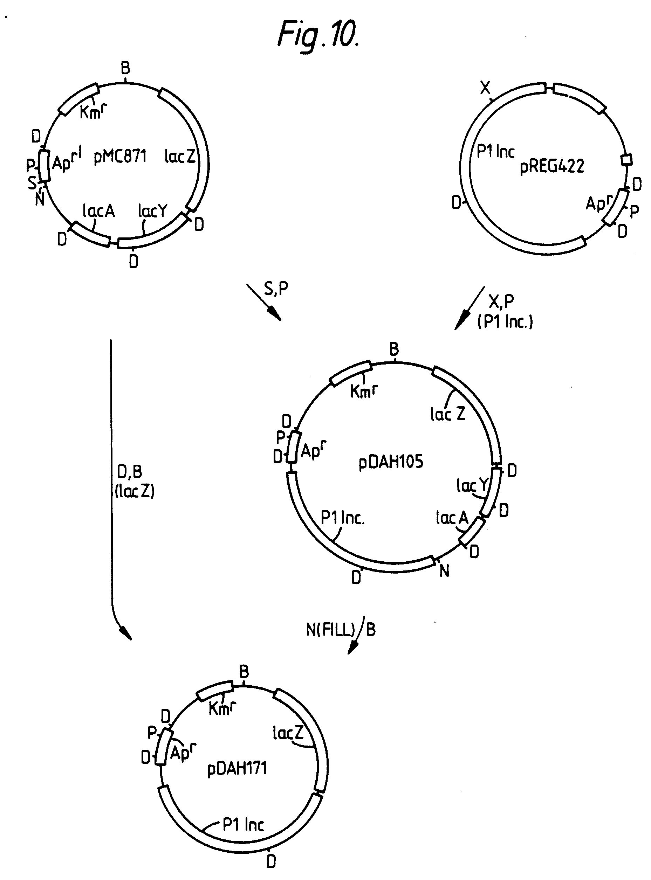

Plasmid pDAH171 is a promoter fusion probe we have constructed for use in M.xanthus. Plasmid pMC871 (Casadaban et al. 1980) was modified by various deletions, the regeneration of the $Ap^r$ gene and the insertion of the P1 Inc. fragment. Figure 10 illustrates construction of pDAH171. The major PstI-SalI fragment of pMC871 (Casadaban et al., 1980) was ligated with the P1 Inc. XhoI-PstI fragment of pREG422. The resultant plasmid pDAH105 could be efficiently transduced with Coliphage P1 and contained a fortuitously regenerated $Ap^r$ determinent. The unnecessary lacA and lacY genes were deleted. Plasmid pDAH105 was cut with NcoI and the ends filled using Klenow fragment (Maniatis et al. 1982) After digesting with BamHI the major fragment was isolated and ligated to the BamHI. DraI fragment of pMC871 encoding the promoterless lacZ gene to form pDAH171. The resulting plasmid pDAH171 (see Figures 5 and 10) contains a unique BamHI site upstream of a promoterless E.coli lacZ gene.

The plasmid pDAH171 was cut with Hind III and BamHI and the BglII. Hind III fragment containing the site of the Ω1911 Tn5 insertion from pDAH201 (Figure 4) was inserted. The resulting plasmid pDAH213 (Figure 5) had only an $Ap^r$ determinant which is of no use in M. xanthus. Therefore, the $Km^r$ determinant of Tn5 from plasmid pDAH141 was inserted. The resultant plasmid pDAH217, which thus contains the light-inducible promoter and lacZ sequences could then be transduced into DK101 and the Co-integrate selected.

**Assay of beta-galactosidase activity**

For qualitative assessment, beta-galactosidase was assayed in two ways. Colonies were picked onto agar containing 40 ug/ml of 5-bromo-4-chloro-3-indolyl-beta-D-galactoside.

This colourless dye, when cleaved by beta-galactosidase, turns blue. Alternatively colonies were sprayed directly with DMSO containing 10mg-ml 4-methylumbelliferyl-beta-D-galactoside. This material, when cleaved, gives rise to 4-methylumbelliferone which fluoresces in UV light.

For quantitative assessment, beta-galactosidase was assayed using 0-Nitrophenyl-beta-D-galactoside by the method described in Miller (1972).

**The Ω1911 region of M.xanthus**

The DNA adjacent to Ω1911 was cloned as described in the methods. The five plasmids obtained; pDAH161, pDAH162, pDAH163, pDAH164 and PDAH165 were subjected to restriction enzyme analysis to produce the restriction site map presented in Figure 3; The orientation of Tn5 in DK2727 and of Tn5-132 in DK6003 was deduced from Southern blot analysis.

Evidence that the mutation leading to orange colony formation in Ω1911 strains is dominant

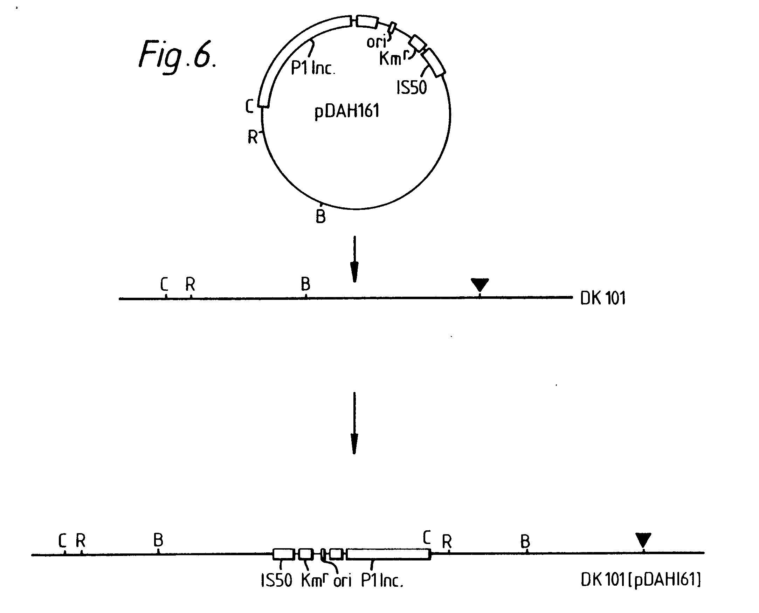

When plasmids pDAH161, pDAH162 and pDAH163 were transferred from E.coli to M.xanthus strain DK101 the resultant colony colour was the same as seen in DK2727. This was surprising as the resultant strains would all contain tandem duplications of the region of M.xanthus cloned in the plasmid (see figure 6 for an example) Plasmids pDAH157, pDAH158 (subclones of pDAH161), pDAH167, pDAH168, pDAH169 and pDAH170 (subclones of pDAH163) all gave the same orange phenotype on transfer. When pDAH164, pDAH165 and pDAH166 (a subclone of pDAH165) were transduced into DK101 the colony colour was unaffected. This result indicated DNA to the left of Ω1911 (as drawn in Figure 3) was the relevant portion.

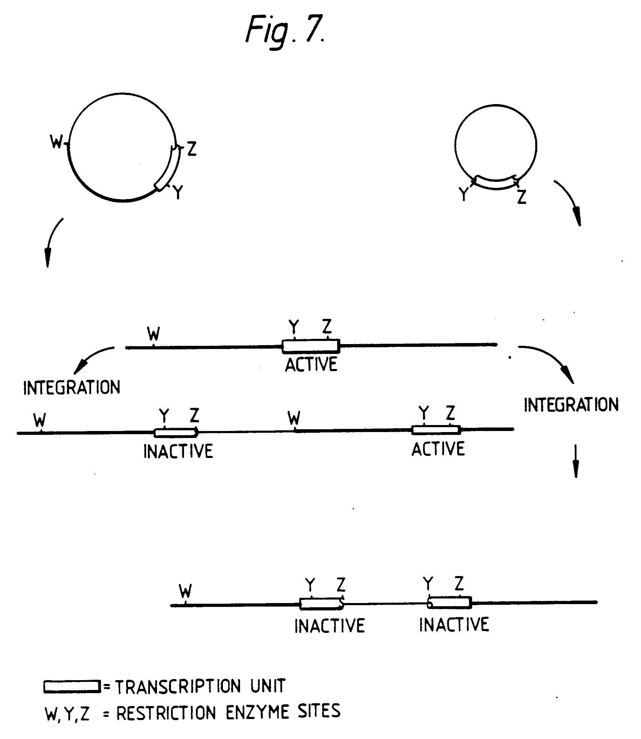

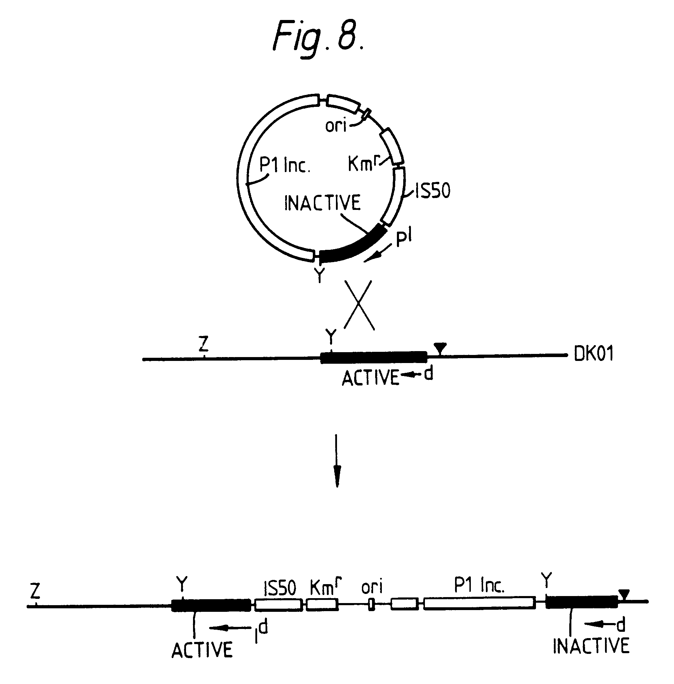

One possible explanation of these results might have been that the DNA to the left of Ω1911 compromised a very large transcriptional unit of greater that 17.6 Kb, i.e. one end was to right of Ω1911 and the other would be to the left of the ClaI Site at 17.6 Kb. The result of integration of such a plasmid would be to uncouple the ends of the tanscription of unit in the chromosomal homologue (see Figure 7) this, in turn, would result in the loss of gene activity. If this were true one would predict that any subclone of the region to the left of Ω1911 would give the same result. This is not the case as the plasmids pDAH191, pDAH193, pDAH202, pDAH203, pDAH204 and pDAH205 (subclones of pDAH163) all gave the yellow colony colour upon transduction. This must imply that Tn5, upon insertion into the Ω1911 site, gave rise to a dominant mutation. The result from pDAH169 etc. indicated the mutant phenotype must be conferred by the

piece or DNA between Ω1911 and the SmaI site of 0.42 Kb. The lack of the orange phenotype upon transduction with pDAH205 implies that the Tn5 itself might be involved. At this point it was learned that strains containing the chromosomal region Ω1910 are orange because they constitutively produce carotenoids which are normally produced only in intense light (Buchard & Dworkin 1966). Southern blot analysis demonstrated that Ω1910 and Ω1911 were indentical. This was not surprising as they originated from the same pool of Tn5 insertions. Using this information it became plausible that Tn5 insertion into Ω1911, rather than having destroyed a gene as one might normally predict, had in fact activated a gene. This would explain how a mutant gene could be dominant to wild type gene in a tandem duplication. One way in which a gene could be activated by an insertion would be if it were normally repressed and the repressor binding site or operator region were destroyed. In this case the repressor would not bind and hence the promoter would be expressed constitutively (see Figure 8). We predicted the presence of a gene to the left of Ω1911 that controls carotenoid production. The promoter is also to the left of Ω1911. The operator, however, which would be upstream of the promoter, has been destroyed by a Tn5 insertion in DK2727. Figure 8 shows the effect of the integration of a mutant, constitutive promoter into a gene containing a "normal" promoter and explains how a fragment internal to the gene but containing the promoter could still lead to a constitutive phenotype upon integration. Once a plasmid containing a gene with a constitutive promoter integrates into the chromosomal homologue of the plasmid insert, a gene will be placed under the control of a constitutive promoter whilst the other copy remains under its 'normal' controls. If the plasmid contains an insert from the promoter to a restriction site within the translational unit (y in the diagram) then integration will result in a full length translational unit connected to the constitutive promoter whilst, the foreshortened copy of the translational unit will be connected to the wild type promoter (not shown). We might predict that pDAH169 and pDAH170 be cases in point considering the small amount of M.xanthus DNA (0.42 Kb) present.

## Possible Mutation of Promoter Mutated By Ω1911 Light Inducible in Wild Type Cells

Carotenoid production is light inducible in wild-type cells and the gene to the left of Ω1911 appears to control carotenoid production. Therefore, the possibility arises that the normal gene is light inducible. To test this possibility we cloned the M.xanthus DNA without the Tn5 insert at Ω1911. This DNA was cloned from DK101 using the novel reversing vector pDAH160 as described in the Methods section. DNA from the resultant plasmid pDAH201 was then used in the construction of a promoter fusion. It was predicted from the analysis of pDAH169 etc., that the promoter must be upstream of the BglII site to left of Ω1911. DNA upstream of the BglII site was therefore coupled, in the correct orientation to a promoterless E.coli lacZ gene (see Methods). The resultant plasmid, after suitable manipulation, was transferred to M.xanthus to form DK101 [pDAH217] (Figure 5). When this strain was exposed to light it produced more beta-galactosidase (the product of the lacZ gene) than did DK101 [pDAH217] cells kept in the dark. It should be noted that dark grown cells produce beta-galactosidase indicating the presence of a constitutive level of expression.

## The mechanism of light induction of the promoter

As stated above, a simple explanation of the constitutive nature of the gene to the left of Ω1911 would be that a repressor binding site had been destroyed by Tn5 insertion in Ω1911 strains. In that case it should be possible to isolate mutants that lack this repressor. The expected phenotype of such repressor mutants should be the same as DK2727, i.e. constitutive carotenoid production. There were three such strains, DK717, DK718 and DK406, present in the Kaiser bacterial strain collection at Stanford. All had been isolated during chemical mutagenesis procedures (see table 1). DK717 was orange, the same as DK2727, however, DK718 and DK406 were very deep orange and produced copious amount of carotenoids in the dark (F.Murillo, personal communication). When plasmid pDAH217 was transduced into DK717, DK718 and DK406, beta-galactosidase was still light inducible in DK717 but it became constitutive in DK718 and DK406. This implied that the latter pair of strains had lost the repressor.

## Precise Measurements of beta-calactosidase Activity in Various Constructs

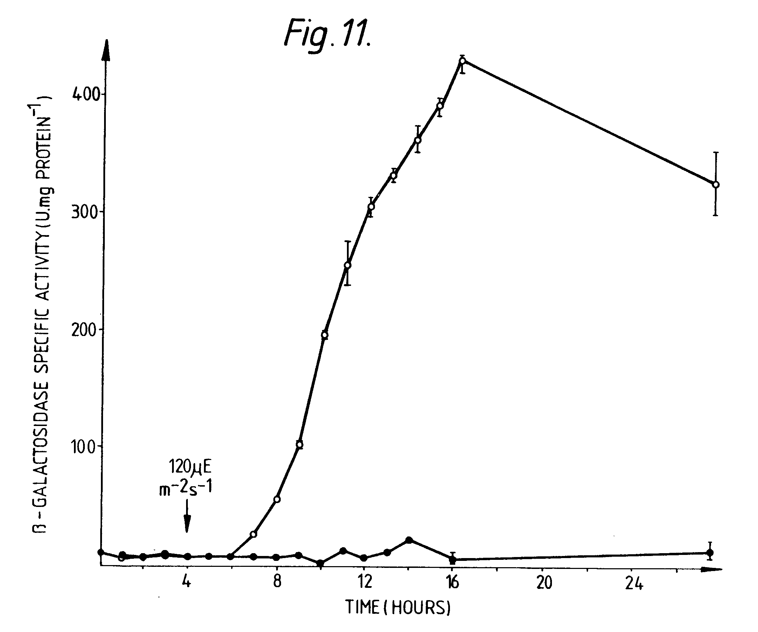

DK101 [pDAH217] (Figure 5) was cultured in the dark and the culture split into two. At the point indicated in Figure 11 one of these cultures were exposed to 120 $\mu$ E m$^{-2}$s$^{-1}$ of light. The other was kept in the dark. Samples were taken and assayed for beta-galactosidase and the results are presented in Figure 11. The culture grown in the light produced nearly 40 times more beta-galactosidase than the one grown in the dark. The light regime chosen was arbitrary and experiments are progressing to investigate if greater induction is possible under different regimes. The experiment was repeated using DK717 [pDAH217], DK718 [pDAH217], DK406 [pDAH217] and DK1050 [pDAH217]. The maximal and minimal levels of beta-galactosidase activity seen were extracted from results presented as in Figure 11 and these are presented in Table 2. DK718 [pDAH217] still retained a two fold light induction of beta-galactosidase activity implying the repressor had not been completely inactivated by the mutation in DK718. DK406 [pDAH217] could not be further induced in light and produced greater than 1000 fold more beta-galactosidase in the light or dark when compared to the uninduced (constitutive levels) of DK1050 [pDAH217]. the observation that beta-galactosidase specific activity increased throughout the growth cycle of both DK406 [pDAH217] implied that beta-galactosidase synthesis was faster than growth rate and production had not reached steady state.

The light induced 17 fold induction of beta-galactosidase in DK717 [pDAH217] compared with the four fold induction in the dark demonstrates that the repressor of the light inducible gene is not affected in DK717 unlike in DK718 and DK406. Possibly the Car$^C$ phenotype is due to a mutation in a gene that is normally regulated by carR but has now become derepressed. The much lower induction by light seen in DK717 [pDAH217] compared to DK101 [pDAH217] most probably reflects masking of the chromophore for light induction by the carotenoids present constitutively in DK717.

### Location of repressor gene

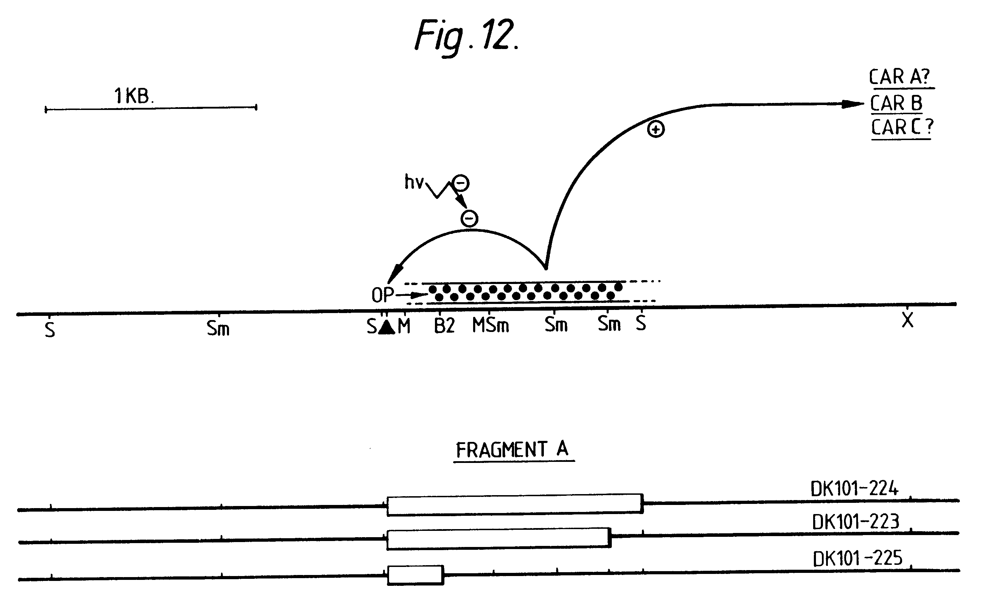

Martinez-Laborda et al. (1986) demonstrated that the lesions in DK406 and DK718 - hence the repressor - mapped close to Ω1910 and hence Ω1911. We therefore transduced pDAH161, pDAH164 and pDAH201(figure 3) into DK406 and DK718. pDAH164 had no effect on the Car$^C$ phenotype. We therefore concluded that the gene for the repressor did not lie to the right ofΩ1911 as drawn in figure 3. The majority of the transductants containing pDAH161 and pDAH201 were light orange in the former and yellow in the latter. The minority stayed red/deep orange. We concluded that the repressor gene was therefore encoded in the DNA to the left of Ω1911 as drawn in figure 11. The minority of red transductants were presumed to be due to gene conversion of the in-coming wild-type repressor gene as seen for other genes(Shimkets et al.,1983). The position of the repressor was narrowed down by transduction of the plasmids of the nested series, shown below the chomosome in figure 3; into strains DX406 and DK718. All of the plasmids tested,including pDAH205, abolished the Car$^c$ phenotypes in the majority of cases. Hence the repressor gene that had been mutated in DK406 and DK718 must be carried on the BglII.SacI fragment labelled "fragment A" in figure 12. Hence the repressor gene is directly downstream of the light- inducible promoter on which it acts.

### Location of the activator of carB

We proposed in our preliminary patent application, GB 8615263,that downstream of the light inducible promoter there had to be an activator of carotenoid synthesis. Balsalobre et al. confirmed the above prediction. Further experiments by ourselves have allowed us to more precisely position the genes encoding the activator.

When pDAH205 was transduced into DK101 the carotenoid production remained light-inducible. As the BglII is downstream of the promoter as shown by the DK101 [pDAH217] experiments (Figure 5) one end of the activator gene must have been removed. Therefore the other end of the activator gene must be confined within the BalII.SacI fragment otherwise integration of pDAH205 would have inactivated the activator gene, as illustrated in Figure 7. Therefore the activator must be encoded at least partially within the BalII.SacI fragment as is the repressor of the light-inducible promoter. This was confirmed by the construction of deletion strains DK101-223, DK101-224 and DK101-225. Plasmids pDAH223, pDAH224 and pDAH225 were constructed by deleting the portions of the chromosome shown in Figure 12 and inserting in their place a drug resistance marker (Kanamycin resistance) bounded by a transcription terminator from coliphage fd. The deletions were inserted into DK101 by transduction of the plasmids pDAH223, pDAH224, pDAH225 followed by gene replacement. Those transductants that had lost the plasmids but retained Kan$^r$ were confirmed by Southern blot analysis. All such transductants were Car$^-$. Therefore loss of the activator is epistatic to the loss of the repressor which is what would be predicted.

### A model for the light induction of car genes in M.xanthus

The position of the activator and repressor and the observed phenotypes of the various mutants and constructs have been assembled into a model of light induction of gene activity as illustrated in figure 12. A repressor binds to the operator (0) site and inhibits promoter activity. This repressor activity is inhibited by light(or its products) via a mechanism at present under study. Once the promoter is activated,transcription can start which leads to the synthesis of more repressor, which will be inactivated in the light,and the activator of carB. The activator and repressor could be the same gene or two separate genes encoded within the SacI fragment. Activator activity is independant of light as shown by the Car$^C$ phenotype in which the car genes are activated in the dark. Once light is no longer present to inactivate the repressor, the car regulon is shut off. Light can be removed by either switching off the source or absorption of the light (or light products) by the newly synthesised carotenoids.

## Cloning of the mutant repressors

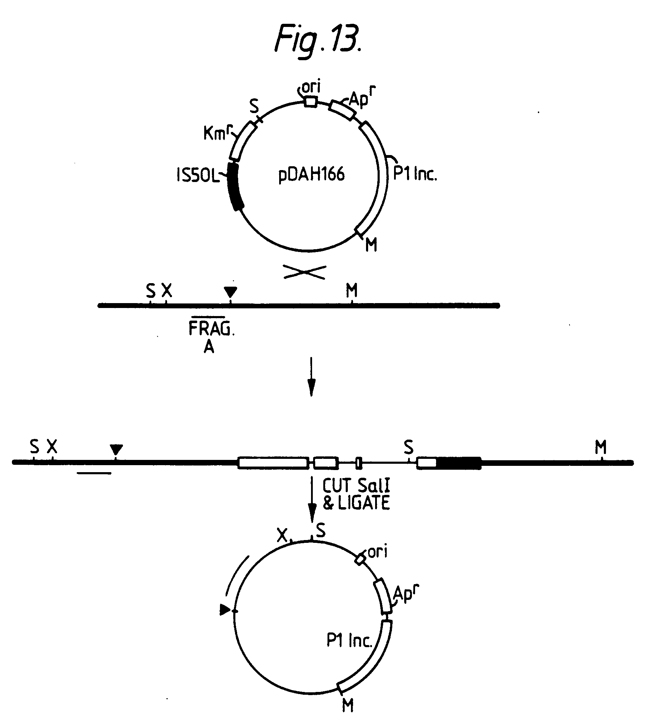

The DNA incorporating the SacI fragment was cloned from DK406 and DK718 as shown in figure 13. We are at present subcloning the DNA to start sequencing studies. The SalI.MluI region was also cloned from DK1050 so that DK406 can be compared to its progenitor.

## Summary of the Drawing Figures

Figure 1 Tn5-132 replacement of Tn5 in DK2727. P1::Tn5-132 was introduced into M.xanthus strain DK2727 as described in the Methods section. Recombination occured between the two homologous insertion sequences, IS50, at the ends of Tn5 and Tn5-132. The IS50's at each end of the transposons are 1.5kb. long and are identical bar one base pair (Auerswald et al.,1980). Following recombination P1::Tn5 is lost leaving DK6003 with the illustrated chromosome structure.

Figure 2 Cloning of DNA adjacent to Ω1911. pREG429 was transduced into DK6003. The plasmid pREG429 could integrate, via homologous recombination either into the left hand IS50 or the right hand IS50. For ease of presentation, integration into only the left hand IS50 is illustrated. These integration events give rise to Km$^r$ colonies. DNA was isolated from a pool of such colonies and subjected to the restriction enzyme digestion and ligation described in the Methods. When ClaI was used on DNA resulting from the integration illustrated here, the only DNA that could give rise to a Km$^r$ colony upon transformation of E.coli would be pDAH161,assuming complete digestion. Digestion with EcoRI gave rise to pDAH162 and with BamHI to pDAH163 (not illustrated.

Figure 3 Restriction map of DNA adjacent to Ω1911. The order and distance of each restriction site is given in kb of DNA with respect to Ω1911. The position of myxococcus DNA present in the various plasmids discussed in the text are illustrated. The vector portions of the plasmids are not included to aid presentation. Where the IS50 was also present on the plasmid adjacent to the M. xanthus DNA it is illustrated. Paired plasmid numbers,i.e. pDAH157 plus pDAH158;pDAH167 plus pDAH168;pDAH169 plus pDAH170; and pDAH203 plus pDAH204 indicate where the M.xanthus DNA was inserted into a vector in both of the two alternative directions.

Figure 4 Cloning of chromosomal DNA lacking the Tn5 insertion. Plasmid pDAH194 was constructed as described in Methods. Upon transduction to DK101 the co-integrate illustrated was formed. Digestion with Hind III and ligation resulted in plasmid pDAH201 which was selected by transformation into E.coli.

Figure 5 Construction of the promoter fusion probe. Studies outlined in the Results section indicated the possibility of a light-inducible promoter to the right of the BglII site as drawn in Fig 3. (indicated as B2). Chromosomal DNA to the right of the BglII fragment ofrom pDAH201 was inserted upsteam of the promoterless E.coli lacZ gene in pDAH171. After reintroduction of a Km$^r$ determinant from pDAH141 the resultant plasmid,pDAH217 was transduced into DK101 to produce DK101[pDAH217]. The ability of light to promote lacZ activity was investigated in strain DK101[pDAH217].

Figure 6 Transduction of cloned DNA back into DK101. Plasmids were transduced into DK101. The resultant pDAH161 co-integrate is illustrated as an example. In each case a tandem duplication of the cloned DNA was formed with the vector sequences at the novel join.

Figure 7 Effect of integration of plasmids containing partial fragments of a transcriptional unit. In the left hand case one complete and one incomplete transcriptional unit will result in which case the former might be expected to complement the latter. In the right hand case the plasmid contains an internal fragment of the transcription unit. Upon integration into a wild type chromosome the transcriptional unit is disrupted in both copies.

Figure 8 Effect of integration of a mutant, constitutive promoter into a gene containing a "normal" promoter.

Figure 9 Construction of the novel "reversing" vector pDAH160.

Figure 10 Construction of the plasmid pDAH171.

Figure 11 Effect of light on beta-galactosidase production by DK101[pDAH217]. A culture of DK101-[pDAH217] cells was split between two flasks which were agitated at 30°C. At the time indicated by the arrow one of the flasks was exposed to 120µE m$^{-2}$s$^{-1}$ of white light,the other was kept in the dark. Triplicate or quadruplicate samples were taken at specified intervals and protein and beta-galactosidase assays were performed. The error bars indicate the spread of beta-galactosidase readings. The circles represent the mean value.O = cells exposed to light ; O = cells kept in the dark.

Figure 12 Model of light activation of carotenoid (car) genes of M.xanthus. It is proposed that there is either a bifunctional gene; or two separate genes encoding a repressor of the promoter and an activator of carB. The repressor function,but not the activator function,is light dependent and is inactivated by light. It is further proposed that the Tn5 insert which inserted at the point marked (the operator) stopped repressor binding such that promoter activity,and hence activator synthesis, became constitutive. Point mutations that are constitutive for carR activity map within "fragment A" . DK101-223,DK101-224 and DK101-225 are strains that contain the in vitro constructed deletions indicated by open boxes. These strains have lost the ability to synthesise carotenoids in the light (Car$^-$).

The abbreviations are as before including O = operator; P = promoter; O = positive regulation; O = negative regulation; and hv = light.

N.B. the orientation has been reversed with respect to figure 3.

Figure 13 Cloning of the mutant repressors of DK406 and DK718 and the repressor of DK1050 . Plasmid pDAH166 (figure 3) was transduced into DK40c,DK718 and DK1050. The chromosomal DNAs of the resultant transductants were isolated and purified on CsCl gradients. These DNAs were then cleaved with SalI, size fractionated,ligated,and transformed into competent E.coli. Those transformants that were Km$^s$ and Ap$^r$ were selected and preparations made of the plasmid DNA. In each case a plasmid was obtained with the predicted structure shown in the lower part of the diagram. As pDAH166 does not contain "fragment A", gene conversion could not have occured during the cloning process. Hence "fragment A's" from DK406,DK718,and DK1050 have been isolated.

## TABLE 1

| Strain | Genotype | Phenotype |
|---|---|---|
| *Myxococcus xanthus* | | |
| DK101 | *sglA1* | partially lacking social motility. original progenitor of starred (*) strains |
| DK717* | *sglA1* *red-1* | orange coloured colonies |
| DK718* | *sglA1* *red-2* | deep orange coloured colonies |
| DK2727* | *sglA1* *cglF1* Ω1911 | orange coloured colonies lacking motility, with a Tn5 (Km$^r$) insert at Ω1911 |
| DK6003* | *sglA1* *cglF1* | Tn5-132 (Tc$^r$) replacement of Ω1911 Tn5 of DK2727 |
| DK1050 | – | wild type *M.xanthus* strain |
| DK406 | *red* | deep orange mutant of DK1050 |
| *P1 coliphage* | | |
| P1 :: Tn5-132 | – | wild type P1 with Tn5-132 (Tc$^r$) insertion |

P1 :: Tn9    clr100                  thermoinducible P1
clr100                               with Tn9 (Cm$^r$)
                                     insertion


E.coli MC1061 (Casadaban & Cohen 1980)

All strains are freely available from the bacterial strain collection of Professor Dale Kaiser, Department of Biochemistry, Stanford University Medical Centre, Stanford, CA94305, U.S.A.

## TABLE 2

Maxima and Minima of beta-galactosidase expression in the light and dark of various strains containing integrated pDAH217.

| Strain | Phenotype | Minima i[*] | Maxima (light[*]) | Maxima (dark[*]) |
|---|---|---|---|---|
| DK101 [pDAH217] | Wild type | 10.89 | 431.28 | 15.05 |
| DK717 [pDAH217] | Car$^C$ derivative of DK101 | 2.53 | 43.52 | 10.22 |
| DK718 [pDAH217] | Car$^C$ derivative of DK101 | 108.4 | 2677.5[ii] | 1347.7[ii] |
| DK1050 | Wild type | 3.71 | 351.55[ii] | 23.06 |

12

[pDAH217]

DK406      Car$^C$      160.6   3401.1$^{ii}$ 4077.9$^{ii}$
[pDAH217]    derivative
           of DK1050

i.     Constitutive level of beta-galactosidase expression of dark grown cells at the start of the experiment.

ii.     The specific activity of beta-galactosidase was still increasing with time when the experiment was terminated.

\*      (Specific activity of beta-galactosidase n U/mg protein).
One unit (U) of beta-galactosidase activity is defined as the amount of enzyme that produces 1 μmole of O-Nitrophenol from 1 μmole of O-Nitrophenyl-beta-D-galactoside in one minute at 37°C.

A culture of E.coli MC1061 harbouring plasmid pDAH217 was deposited in accordance with the terms of the Budapest Treaty at the National Collection of Industrial Bacteria, Aberdeen, Research Station, P.O. Box 31, 135 Abbey Road, Aberdeen, Scotland AB9 8DG on 20th June, 1986, and has been accorded the accession number N.C.I.B. 12269.

A culture of E.coli. ED8812 harbouring plasmid pDAH201 was deposited in accordance with the terms of the Budapest Treaty at the National Collection of Industrial Bacteria on 19th June, 1987, and has been accorded the accession number NCIB 12493. E.coli. ED8812 is thr, leu, thi, lacY, lacZ(M15), tonA, supE, hsdR, hsdM, S⁻, lambda⁻, lambdaˢ, and is freely available from the culture collection of Edinburgh University, Scotland, United Kingdom.

Abbreviations used in the Figures and Specification

P1 Inc.            —    P1 Incompatability region
                        promotes efficient P1
                        transduction)

ori               —    Origin of replication in
                        E.coli

$Km^r/Km^s$        —    Kanamycin resistance/sensitivity

$Tc^r/Tc^s$        —    Tetracycline resistance/
                        sensitivity

$Ap^r/Ap^s$        —    Ampicillin resistance/
                        sensitivity

$Cm^r/Cm^s$        —    Chloramphenicol resistance/
                        sensitivity

IS50              —    IS50 insertion sequence

lacZ              —    Promoterless E.coli
                        lacZ gene

                  —    Site of Tn5 in Ω1911

B                 —    BamHI site

B2                —    BclII site      D  —  Dra I site

C                 —    ClaI site       N  —  Nco I site

H3                —    Hind III site   P  —  Pst I site

R                 —    EcoRI site      R5 — EcoRV site

14

S              –        SalI site       $Ap^{r^1}$ – Part of $Ap^r$ gene

Sm         –        SmaI site

X            –        XhoI site

M            –        MluI

ml         –        millilitre

µg         –        microgram

µl         –        microlitre

kb         –        Kilobase pairs

REFERENCES

Auerswald, E.A., G. Ludwig and Schaller (1980) Cold Spring Harbor Symp. Quant. Biol. 45:107-113.

Balsalobre, J. M., Ruiz-Vazquez, R. M. and Murillo, F. J. (1987) Proc. Natl. Acad. Sci. USA. 84,2359-2362

Berg, D. E., C. Egner, B. J. Hirschel, J. Howard, L. Johnsrud, R.A. Jorgensen and T. D. Tisty (9180) Cold Spring Harbor Symp. Quant. Biol. 45:115-123.

Buchard, R.P. and M. Dworkin (1966) J. Bacteriol. 91:535-545.

Casadaban M. J. and S. N. Cohen (1980) J. Mol. Biol. 138:179-207.

Casadaban, M. J., J. Chow and S. N. Cohen (1980) J. Bacteriol. 143:971-980.

Dworkin, M. and D. Kaiser (1985) Science 230:18-24.

Hodgkin, J. and D. Kaiser (1977) Proc. Acad. Natl. Sci. USA 74:2938-2942.

Jorgensen, R.A., S. J. Rothstein and W. S. Reznikoff (1985) Molec. Gen. Genet. 177, 65-72.

Kroos, L. and Kaiser, A. D. (1983) Proc. Natl. Acad. Sci. USA 81,5816-5820.

Kuner, J. M., L. Avery, D. E. Berg and D. Kaiser (1981) In Schlessinger, D. (ed) Microbiology 1981 American Soc. for Microbiol., Washington D.C., p. 128-132.

Maniatis, T., E. F. Frisch and J. Sambrook (1982). Molecular Cloning. Cold Spring Harbor Laboratory, Cold Spring Harbor, N.Y.

Martinez-Laborda, A., Ellis, M., Ruiz-Vasquez, R. and Murillo, F. J. (1986) Molec. Gen. Genet. 205,107-114.

Miller, J. (1977) Experiments in Molecular Genetics. Cold Spring Harbor Laboratory, Cold Spring Harbor, N.Y.

Rosenberg, E. (ed) (1984) Myxobacteria-Development and Cell Interactions Springer-Verlag, New York Inc., New York, N.Y.

Shimkets, L. J., R. E. Gill and D. Kaiser (1983) Proc. Acad. Natl. Sci. USA 80:1406-1410.

Sodergren, E. and Kaiser, D. (1983) J. Mol. Biol. 167 295-310.

**Claims**

1.    A recombinant DNA sequence comprising a light inducible promoter sequence which is the light inducible promoter sequence of plasmid pDAH217 (NCIB12269) or pDAH201 (NCIB12493) or mutants of that sequence which function as a light inducible promoter.

**2.** A recombinant DNA sequence according to claim 1 comprising a second DNA sequence coding for a polypeptide to be expressed, the second DNA sequence not being operatively linked to the promoter in nature.

**3.** A recombinant DNA sequence according to claim 1 or 2 comprising a restriction site in which the second DNA sequence is inserted.

**4.** A recombinant DNA sequence according to any preceding claim which comprises an expression vector.

**5.** A host organism transformed by a recombinant DNA sequence according to any preceding claim.

**6.** A host according to claim 5 which is a bacteria.

**7.** A host according to claim 6 which is E. coli or M. xanthus.

**8.** A method of producing a polypeptide which comprises:
a. inserting a DNA sequence coding for the polypeptide into a recombinant DNA sequence according to any one of claims 1 to 4 to place it under the operative control of the light inducible promoter and to form a recombinant DNA molecule;
b. transforming a competent host with the said recombinant DNA molecule; and
c. subjecting the host to light to induce the light inducible promoter whereby the gene is expressed.

**9.** Plasmid pDAH217 (NCIB12269) or pDAH201 (NCIB12493).

**10.** A method according to claim 8 or 9 in which the host is a bacteria.

**11.** A method according to claim 10 in which the host is E. coli or M. xanthus.

**12.** A method according to claim 11 in which the host is E. coli MC1061 or M. xanthus DK101.

**Patentansprüche**

**1.** Rekombinante DNA-Sequenz, umfassend eine lichtinduzierbare Promotorsequenz, die die lichtinduzierbare Promotorsequenz des Plasmids pDAK217 (NCIB12269) oder pDAH201 (NCIB12493) ist oder Mutanten dieser Sequenz, die als ein lichtinduzierbarer Promotor wirken.

**2.** Rekombinante DNA-Sequenz nach Anspruch 1, umfassend eine zweite DNA-Sequenz, die ein zu exprimierendes Polypeptid kodiert, wobei in der Natur die zweite DNA-Sequenz mit dem Promotor nicht funktionsfähig verknüpft ist.

**3.** Rekombinante DNA-Sequenz nach Anspruch 1 oder 2, umfassend eine Restriktionsstelle, an der die zweite DNA-Sequenz eingefügt ist.

**4.** Rekombinante DNA-Sequenz nach einem der vorhergehenden Ansprüche, die einen Expressionsvektor umfaßt.

**5.** Wirtsorganismus, der mittels einer rekombinanten DNA-Sequenz nach einem der vorhergehenden Ansprüche transformiert ist.

**6.** Wirt nach Anspruch 5, dadurch gekennzeichnet, daß er ein Bakterium ist.

**7.** Wirt nach Anspruch 6, dadurch gekennzeichnet, daß er E. coli oder M. xanthus ist.

**8.** Verfahren zum Herstellen eines Polypeptids, umfassend
a. das Einfügen einer das Polypeptid kodierenden DNA-Sequenz in eine rekombinante DNA-Sequenz nach einem der Ansprüche 1 bis 4, um es unter die operative Kontrolle des lichtinduzierbaren Promotors zu bringen und ein rekombinantes DNA-Molekül zu bilden;
b. das Transformieren eines tauglichen Wirts mit dem rekombinanten DNA-Molekül; und

16

EP 0 310 619 B1

c. das Einwirkenlassen von Licht auf den Wirt, um den lichtinduzierbaren Promotor zu induzieren, wodurch das Gen exprimiert wird.

**9.** Plasmid pDAH217 (NCIB12269) oder pDAH201 (NCIB12493).

**10.** Verfahren nach Anspruch 8 oder 9, wobei der Wirt ein Bakterium ist.

**11.** Verfahren nach Anspruch 10, wobei der Wirt E. coli oder M. xanthus ist.

**12.** Verfahren nach Anspruch 11, wobei der Wirt E. coli MC1061 oder M. xanthus DK101 ist.

**Revendications**

**1.** Une séquence d'ADN recombinant comprenant une séquence de promoteur susceptible d'être induite par la lumière, qui est la séquence de promoteur du plasmide pDAH217 (NCIB12269) ou pDAH201 (NCIB12493) ou des mutants de cette séquence qui fonctionnent comme promoteur susceptible d'être induite par la lumière.

**2.** Une séquence d'ADN recombinant selon la revendication 1, qui contient une seconde séquence d'ADN qui code pour un polypeptide qui doit être exprimé, la seconde séquence d'ADN n'étant pas reliée fonctionnellement au promoteur dans l'ordre.

**3.** Une séquence d'ADN recombinant selon la revendication 1 ou 2, qui comprend un site de restriction dans lequel la seconde séquence d'ADN est insérée.

**4.** Une séquence d'ADN recombinant selon chaque revendication précédente qui contient un vecteur d'expression.

**5.** Un organisme hôte transformé par une séquence d'ADN recombinant, selon chaque revendication précédente.

**6.** Un hôte selon la revendication 5 qui est une bactérie.

**7.** Un hôte selon la revendication 6 qui est E. Coli ou M. Xanthus.

**8.** Une méthode de production d'un polypeptide comprenant :
a) l'insertion d'une séquence d'ADN qui code pour le polypeptide dans une séquence d'ADN recombinant selon l'une quelconque des revendications 1 à 4 pour la placer sous le contrôle fonctionnel du promoteur inductible par la lumière ;
b) la transformation d'un hôte compétent avec cette molécule d'ADN recombinant ; et
c) la soumission de l'hôte à la lumière pour induire le promoteur susceptible d'être induit par la lumière par lequel le gène est exprimé.

**9.** Plasmide pDAH217 (NCIB12269) ou pDAH201 (NCIB12493).

**10.** Une méthode selon la revendication 8 ou 9, dans laquelle l'hôte est une bactérie.

**11.** Un méthode selon la revendication 10 dans laquelle l'hôte est E. Coli ou M. Xanthus.

**12.** Une méthode selon la revendication 11 dans laquelle l'hôte est E. Coli MC1061 ou M. Xanthus DK101.

17

# Fig.1.

EP 0 310 619 B1

# Fig. 2.

*Fig. 3(A).*

Fig. 3(B).

21

# Fig.4.

Fig.5.

Fig.6.

# Fig. 7.

INTEGRATION

ACTIVE

INTEGRATION

INACTIVE

ACTIVE

INACTIVE

INACTIVE

◻▭▭▭◻ = TRANSCRIPTION UNIT
W,Y,Z = RESTRICTION ENZYME SITES

Fig. 8.

Fig. 9.

# Fig.10.

Fig.11.

# Fig.12.

1KB.

CAR A?
CAR B
CAR C?

hv

OP→

S    Sm    S▲M  B2  MSm    Sm    Sm S    X

FRAGMENT A

DK101-224

DK101-223

DK101-225

EP 0 310 619 B1

# Fig.13.